# EUROPEAN PATENT SPECIFICATION

(11) **EP 1 845 835 B1**
(45) Date of publication and mention of the grant of the patent: **19.11.2014**
(21) Application number: 06701745.9
(22) Date of filing: 26.01.2006
(51) Int. Cl.: A61B 1/04, A61B 1/055, A61B 1/05

(54) **ENDOSCOPE WITH MINIATURE IMAGING ARRANGEMENT**
ENDOSKOP MIT MINIATURABBILDUNGSANORDNUNG
ENDOSCOPE AVEC DISPOSITIF IMAGEUR MINIATURE

(30) Priority: 27.01.2005 US 647036 P
(43) Date of publication of application: 24.10.2007
(73) Proprietor: Covidien LP, Mansfield, MA 02048 (US)
(72) Inventor: GILBOA, Pinhas, 34409 Haifa (IL)
(74) Representative: KIPA AB
(86) International application number: PCT/IL2006/000113
(87) International publication number: WO 2006/080015

(56) References cited:
- WO-A1-01/19235
- JP-A- 2000 075 218
- US-A- 5 021 888
- US-A- 6 134 003
- US-B1- 6 381 490
- US-B1- 6 422 994
- US-B1- 6 485 413
- US-B1- 6 547 722

## Description

### FIELD AND BACKGROUND OF THE INVENTION

The present invention relates to endoscopes and, in particular, it concerns a miniature imaging sensor for use with particularly small diameter endoscopes.

It is known to employ endoscopes with imaging sensors to obtain images of body cavities including, but not limited to, the lungs, the stomach, the colon, and the abdomen. Endoscopes for imaging cavities within the lungs are typically referred to as "bronchoscopes", and those for imaging within the colon are typically referred to as "colonoscopes". All such devices with imaging capabilities for examining the inside of body cavities are referred to herein generically as "endoscopes". Until recently, flexible endoscopes employed optical fibers to deliver the image from the distal endoscope tip to its proximal end. In recent years, video endoscopes were built, where a video camera is placed at the distal tip and the image is delivered to its proximal end via electrical wires. This arrangement improves the picture quality and makes the endoscope more flexible, since the electric wires are more flexible than the fiberscopes.

Usually the video camera has an automated gain control (AGC) that controls the exposure duration in order to avoid saturation. The AGC can be implemented internally, occupying some physical area, or alternatively the AG can be controlled from the outside via command lines. For extremely miniature sensors, i.e., with diameters below 3 millimeters, the latter is the only possible solution. The control signals need to be fed into the image sensor via dedicated lines, in addition to other lines that are required for power and video out. Therefore, the minimum number of lines required is: at least two lines for power, two lines for video output and at least one control line, giving a total of no less than 5 lines. Where active illumination is performed by light emitting elements associated with the endoscope tip, this requires an additional two lines. If three-color illumination is used, an additional four lines are required.

An endoscope includes its own light source to illuminate a scene viewed from its tip. The light typically radiates in spherical waves in which the flux density (the power per unit area) drops as the area of the sphere increases. When this is the only source of illumination, the intensity of the light illuminates the objects as a function of the inverse of the square of the distance between the source and the objects. Imaging small intrabody cavities such as small bronchial tubes requires a large dynamic sensing range because of the big difference in distances between the adjacent tissue and the relatively far distance seen at the center of this tube. Practically, since the dynamic range of the sensor is finite, in a wide viewing angle, where very close and very far tissues are seen in the same exposure, it is impossible to get an image that is free of saturation and at the same time clearly shows the dark elements of the scene. A short exposure is preferred for acquiring the image of the adjacent tissue, while more distant tissue requires a long exposure.

The incorporation of light sources into the distal tip of a very miniature endoscope often presents problems of uneven light distribution. In particular, where different colors of illuminating light are supplied from different light emitting diodes (LEDs), or via separate optic fibers from an external source, the differing geometrical positions of the light sources for the different colors often causes color imbalance between different parts of the image. A further problem in very miniature systems is the proximity of the light source to the image detector array which may lead to light leakage between the lens arrangement and the image sensor array.

There is therefore a need for a miniature endoscope which would achieve effective dispersion of illumination, reduce the number of wire connections required to the image sensor chip, and thereby facilitate implementation of an endoscope of diameter no greater than about 2 millimeters.

JP 2000075218 discloses an endoscope including a lens barrel, which holds objective lenses and is formed with an external thread part and fitting part on the outer peripheral part, and an element holding cylinder, which holds a solid-state image pickup element.

US 6,547,722 discloses an endoscope including an optical unit having at least one optical member, and optical unit supporting frame for supporting the optical unit so that its position can be adjusted in the direction of the optical axis

### SUMMARY OF THE INVENTION

The present invention is an endoscope with a miniature imaging arrangement according to the appended claims.

According to the teachings of the present invention there is provided, an endoscope comprising: (a) an elongated flexible body having a distal tip portion; and
(b) an imaging arrangement associated with the distal tip portion, the imaging arrangement including: (i) an image sensor chip including a two-dimensional array of light-sensitive pixels; and (ii) a lens arrangement deployed for focusing light from a field of view onto the image sensor chip so as to generate an image of a scene viewed from the distal tip portion, wherein the lens arrangement is directly affixed to the image sensor chip by a quantity of transparent adhesive.

According to a further feature of the present invention, the lens arrangement includes a cylindrical graded-index lens. Alternatively, the lens arrangement includes a compound lens assembly.

According to a further feature of the present invention, the lens arrangement has a field of view of at least about 60°, and more preferably at least about 90°.

According to a further feature of the present invention, an area of the two-dimensional array of light-sensitive pixels is no more than half a square millimeter.

According to a further feature of the present invention, the imaging arrangement has a diameter of no more than 2 millimeters.

According to a further feature of the present invention, there is also provided: (a) at least one light source for illuminating the scene viewed from the distal tip portion; and (b) an optically dispersive medium distally overlying the light source such that the optically dispersive medium is effective to disperse illumination from the light source, thereby illuminating the scene viewed from the distal tip portion, without obscuring light reflected from the scene from reaching the lens arrangement.

According to a further feature of the present invention, the lens arrangement extends distally beyond the at least one light source, and wherein the optically dispersive medium surrounds the lens arrangement without overlying the lens arrangement.

According to a further feature of the present invention, the imaging arrangement further includes a substantially opaque medium deployed at least between the light source and the two-dimensional array of light-sensitive pixels without obscuring propagation of illumination from the light source towards the scene.

According to a further feature of the present invention, the imaging arrangement further includes a substantially transparent medium overlying both the optically dispersive medium and the lens arrangement.

According to a further feature of the present invention, the at least one light source is implemented as a plurality of light sources of different colors.

According to a further feature of the present invention, the image sensor chip is rectangular, and wherein the plurality of light sources are deployed along no more than two edges of the rectangular chip, the two-dimensional array of light-sensitive pixels being located proximal to a corner of the image sensor chip furthest from the two edges of the rectangular chip.

According to a further feature of the present invention, the at least one light source and the image sensor chip are deployed on a common circuit board.

According to a further feature of the present invention, the circuit board fits within a circular cross-section of diameter 2 millimeters.

According to a further feature of the present invention, there are also provided a plurality of wires passing along the elongated flexible body for connection to the image sensor chip and the at least one light source, the wires being connected to contact regions of the circuit board on a proximal side of the circuit board.

According to a further feature of the present invention, the image sensor chip is connected to exactly four of the plurality of wires.

According to a further feature of the present invention, there is also provided a position sensor arrangement including a plurality of sensor coils, the position sensor arrangement being deployed within the elongated flexible body near a proximal side of the circuit board.

There is also provided according to the teachings feature of the present invention, an endoscope comprising: (a) an elongated flexible body having a distal tip portion; and (b) an imaging system associated with the elongated flexible body, the imaging system including: (i) an image sensor chip including a two-dimensional array of light-sensitive pixels, the image sensor chip being associated with the distal tip portion; (ii) a controller associated with a proximal part of the elongated flexible body, the controller being electrically associated with the image sensor chip via no more than two communication wires extending along the elongated flexible body, wherein the image sensor chip is configured to be responsive to a timing signal generated by the controller to perform a read cycle of the two-dimensional array of light-sensitive pixels in a rolling-shutter mode and to transmit a single frame of image data to the controller, wherein both the timing signal and the image data are transmitted via the no more than two communication wires.

According to a further feature of the present invention, the timing signal is a frame request signal, and wherein the image sensor chip is configured to wait after transmitting the single frame of image data until receiving a subsequent frame request signal from the controller.

According to a further feature of the present invention, the controller is configured to actuate the image sensor chip to generate pairs of similar frames with different exposure durations, the controller being further configured to co-process the pairs of similar frames to derive an enhanced frame having a dynamic range greater than each of the pair of similar frames.

According to a further feature of the present invention, there is also provided an illumination system deployed for illuminating a scene viewed from the distal tip portion, the illumination system being configured for selectively illuminating the scene with each of three different colors of visible light, wherein the illumination system is controlled by the controller such that the controller derives an enhanced frame from a pair of similar frames with different exposure durations sampled for each of the three different colors, the controller being further configured to combine the enhanced frames to generate a color image.

According to a further feature of the present invention, there is also provided an illumination system including at least one light emitting diode associated with the distal tip portion, the light emitting diode and the image sensor chip being mounted on a common circuit board.

According to a further feature of the present invention, there is also provided a quantity of an optically dispersive medium overlying the at least one light emitting diode so as to disperse illumination from the light emitting diode.

### BRIEF DESCRIPTION OF THE DRAWINGS

The invention is herein described, by way of example only, with reference to the accompanying drawings, wherein:
FIG. 1 is a schematic view of an endoscope, constructed and operative according to the teachings of the present invention;
FIG. 2 is an enlarged schematic isometric view of a distal tip portion of the endoscope of Figure 1 with an outer cover removed to reveal an imaging arrangement, constructed and operative according to the teachings of the present invention;
FIG. 3 is a further enlarged schematic isometric view of the imaging arrangement of Figure 2;
FIG. 4 is a partially exploded isometric view of the imaging arrangement of Figure 2;
FIG. 5 is a schematic cross-sectional view taken through the imaging arrangement of Figure 2 illustrating a layered structure of encapsulation of the imaging arrangement according to a further feature of the present invention;
FIG. 6A is a schematic isometric view of an apparatus for use in assembly of the imaging arrangement of Figure 2, the apparatus being shown during a chip alignment step;
FIG. 6B is an enlarged view of a region of Figure 6A designated "B";
FIG. 6C is a schematic isometric view of the apparatus of Figure 6A shown during a lens attachment step;
FIG. 7 is a graphic representation of a relation between pixel output signal and scene brightness for two different durations of exposure, labeled "T₁" and "T₂";
FIG. 8 is a graphic representation of a relation between pixel output signal and scene brightness derived from a pair of exposures of two different durations of exposure as illustrated in Figure 7;
FIG. 9 is a schematic representation of the layout of a CMOS imaging sensor chip from the imaging arrangement of Figure 2;
FIG. 10 is a functional representation of the operation of a CMOS image sensor pixel element from the chip of Figure 9;
FIG. 11 is a schematic representation of a first communication arrangement for bidirectional communication with the imaging sensor arrangement of Figure 2 according to the teachings of the present invention; and
FIG. 12 is a schematic representation of a second communication arrangement for bidirectional communication with the imaging sensor arrangement of Figure 2 according to the teachings of the present invention.

### DESCRIPTION OF THE PREFERRED EMBODIMENTS

The present invention is an endoscope with a miniature imaging arrangement.

The principles and operation of endoscopes according to the present invention may be better understood with reference to the drawings and the accompanying description.

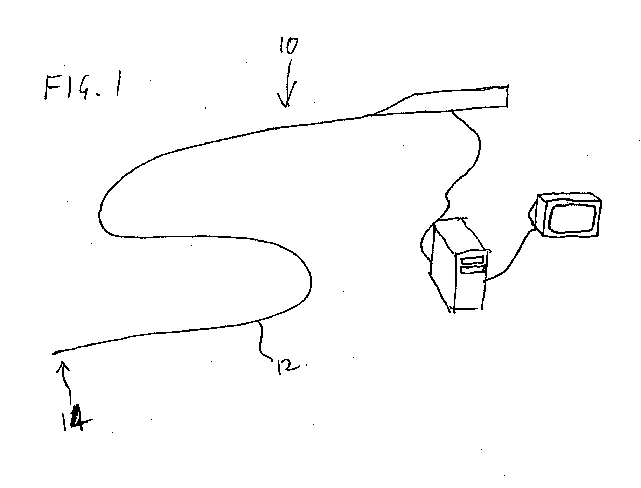

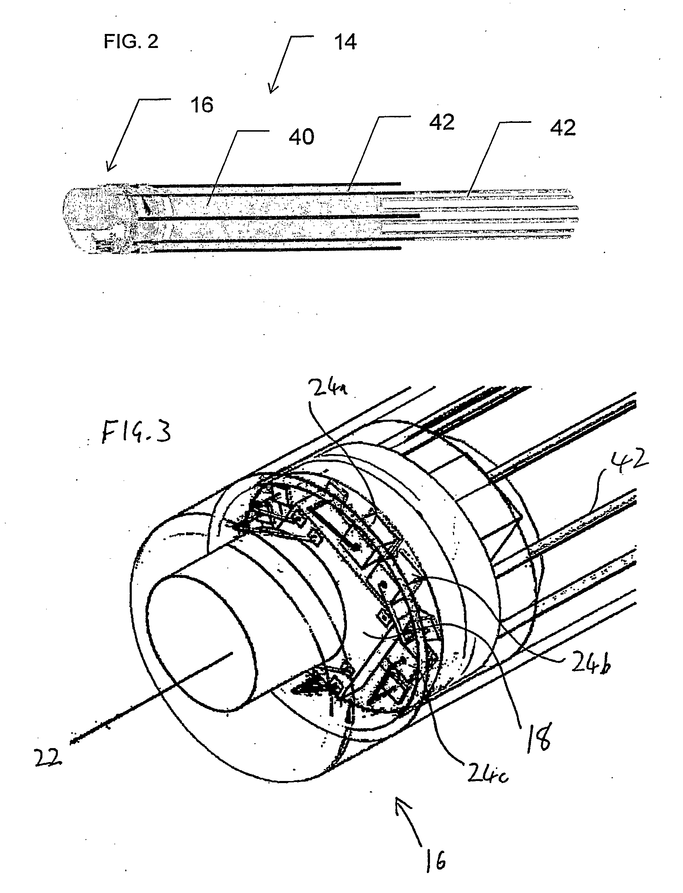

Referring now to the drawings, Figure 1 shows a general view of an endoscope, generally designated **10,** constructed and operative according to the teachings of the present invention. The endoscope has an elongated flexible body **12** with a distal tip portion **14.** As seen in Figures 2-5, an imaging arrangement **16** is associated with distal tip portion **14.** Imaging arrangement **16** includes an image sensor chip **18** including a two-dimensional array **20** of light-sensitive pixels, and a lens arrangement **22** deployed for focusing light from a field of view onto image sensor chip **18** so as to generate an image of a scene viewed from the distal tip portion.

It is a particularly preferred feature of the present invention that elongated flexible body **12** in general, and imaging arrangement **16** in particular, is a small caliber device, preferably of outer diameter no more than 3 millimeters, and most preferably of outer diameter no more than about 2 millimeters. This allows the endoscope to be introduced into small body cavities and passages which are normally inaccessible to conventional endoscopes of larger dimensions. For example, according to certain preferred embodiments, the endoscope may be inserted via a working lumen of a conventional bronchoscope and advanced into bronchial airways beyond the reach of the conventional bronchoscope where conventional procedures would require working "blind". The miniaturization of the imaging arrangement of an endoscope to such small dimensions poses a number of significant problems of implementation. The present invention relates primarily to effective solutions for a number of such problems.

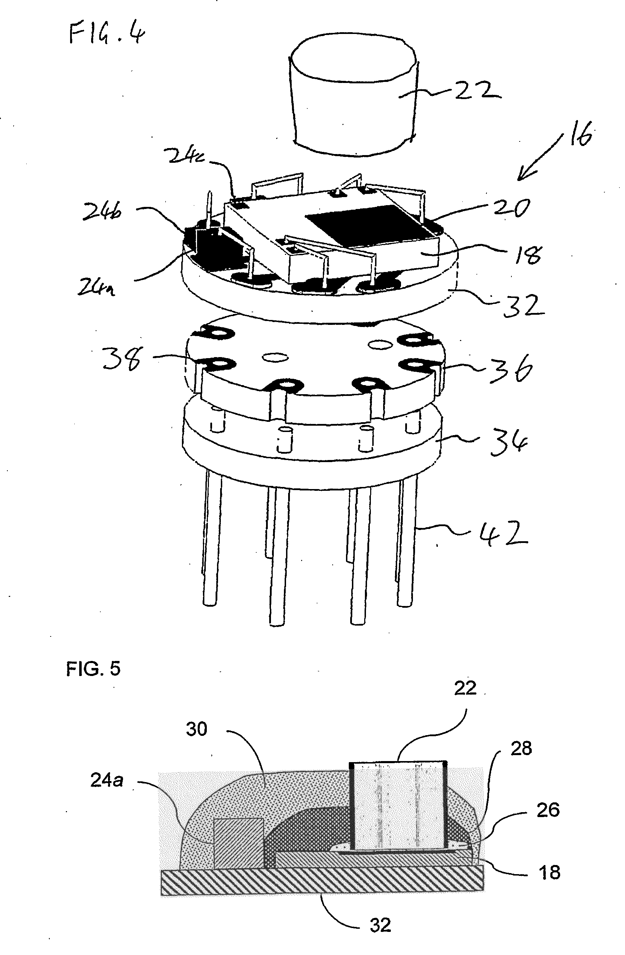

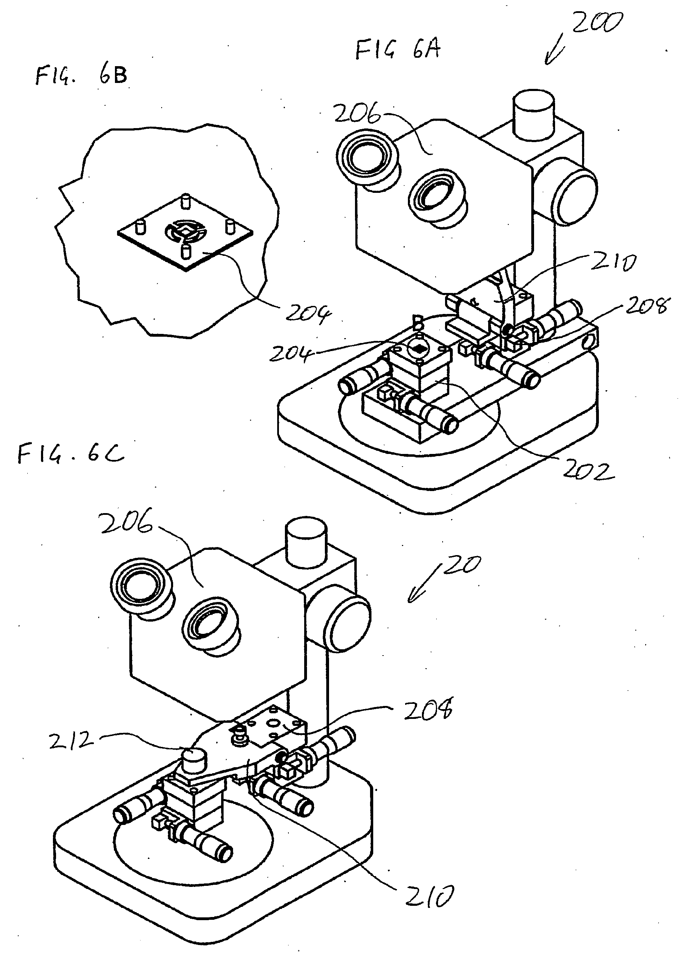

Specifically, one issue plaguing such miniature implementations of an imaging sensor is how to achieve and maintain correct alignment of lens arrangement 22 with sensor array 20 where both the lens arrangement and the sensor chip have dimensions of the order of a millimeter or less. According to one aspect of the present invention, this issue is addressed by directly affixing lens arrangement **22** to image sensor chip **18** by a quantity of transparent adhesive **26** (Figure 5, shown with slight excess at the sides of the lens). According to a further complementary aspect of the present invention, there is provided an apparatus (Figures 6A-6C) for facilitating affixing of lens arrangement **22** to image sensor chip **18** in correct alignment with array **20.**

A further problem plaguing miniature implementations of an imaging sensor is unwanted or uneven distribution of illuminating radiation. Endoscope **10** includes at least one source of illumination, such as light emitting diodes ("LEDs") **24*a***, **24*b*** and **24*c*.** Due to the proximity of the light sources to the sensor array, light may leak around the base of lens arrangement **22,** thereby degrading image quality. Furthermore, particularly where separate colored light sources are deployed asymmetrically relative to the lens arrangement, illumination tends to be non-uniform across the viewed scene and dissimilar between the different colors, leading to color imbalance in the output image. According to one further aspect of the present invention, the issue of light leakage is addressed as illustrated in Figure 5 by deploying a substantially opaque medium **28** between the light sources **24*a***, **24*b*** and **24*c*** and the two-dimensional array **20** of light-sensitive pixels without obscuring propagation of illumination from the light sources **24*a***, **24*b*** and **24*c*** towards the scene to be viewed. According to a second further aspect of the present invention, the problem of non-uniform light distribution is addressed, also as illustrated in Figure 5, by deploying an optically dispersive medium **30** distally overlying light sources **24*a***, **24*b*** and **24*c*** such that medium **30** is effective to disperse illumination from the light source, thereby illuminating the scene viewed from the distal tip portion, without obscuring light reflected from the scene from reaching lens arrangement **22.**

A further issue problematic for the miniaturization of endoscope **10** is the number of connection wires which need to be attached to imaging arrangement **16.** According to a still further aspect of the present invention, the number of connections to image sensor chip **18** is reduced to a total of four: two power connections and two communication connections. For this purpose, the present invention provides both a system and a method for operating an image sensor system in which two communication connections are used bi-directionally for both a frame request to the imaging arrangement and for outputting data from the imaging arrangement.

These and other aspects of the present invention will be better understood from the following detailed description.

Before addressing features of the present invention, it will be helpful to define certain terminology as used herein in the description and claims. Firstly, reference is made to "light" and "illumination". These terms are used herein to refer generically to all parts of the electromagnetic spectrum which can be detected by low-cost silicon-based image sensors, such as CMOS sensors. This includes all of the range of wavelengths from near-ultraviolet through to near-infrared (wavelengths of between 0.25 microns and 1.1 microns). Most preferably, visible light in the range of wavelengths from about 0.4 microns to about 0.75 microns is used. The light may be monochromatic, or may contain a number of different colors simultaneously or alternately. Broad spectrum white light may also be used. Certain particularly preferred options for illumination will be discussed below.

The term "light source" is used herein to refer to any component which releases light from imaging arrangement 16 towards the scene to be viewed. The source may either generate light, as in the case of a LED, or may convey light from a remote location, as in the case of an optic fiber conveying light from a source associated with a proximal part of the endoscope body.

The scene viewed by imaging arrangement **16** may be any scene visible from the distal tip portion **14** of the endoscope. Typically, the invention is implemented as a forward-looking imaging arrangement where the optical axis of lens arrangement **22** is roughly parallel to a central axis of distal tip portion **14.** It should be noted, however, that other implementations, such as a side-looking endoscope, also fall within the scope of the present invention.

Turning now to the features of imaging arrangement **16** in more detail, according to a first preferred option, lens arrangement **22** includes a cylindrical graded-index ("GRIN") lens. Alternatively, a miniature compound lens assembly made from injection molded polymer components (typically polycarbonate) is used. In either case, lens arrangement **22** is preferably cylindrical with a total height of no more than about 1.5 millimeter and a diameter of no more than about one millimeter. The field of view of lens arrangement **22** is preferably at least about 60°, and most preferably at least about 90°.

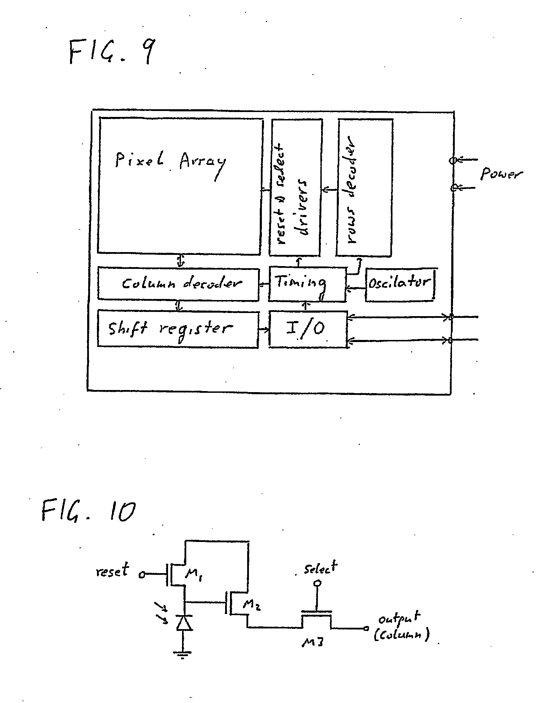

Image sensor chip **18** is preferably a CMOS chip with major dimensions of roughly one millimeter square. Roughly half of the surface area (e.g., a square of side roughly 0.7 mm) accommodates the sensor array **20** while the remaining surface is used for the associated electronic components for reading the array, shown schematically in Figure 9, as is known in the art. Thus, the area of two-dimensional array **20** is typically no more than about half a square millimeter (i.e., 5×10⁻⁷ m²). It has been found that these dimensions, with current production technology commercially available, are sufficient for implementing a monochrome sensor array of resolution approximately 100×100 pixels.

Each pixel is structured from the classical three-transistor architecture as described in Figure 10. Switch M1 is effective to reset the pixel and to charge the photodiode. Impinging light then discharges the diode and creates a voltage difference compared to the reset level. The photodiode voltage is read out via transistor M2 that acts as a source follower. The output voltage is fed into the column readout. The sensor operates in a 'rolling shutter' mode, i.e., each row in turn is reset during reading of that row. The rest of the components of image sensor chip **18** illustrated in Figure 9 are the necessary peripheral logic to read out the array. The control logic includes a vertical address decoder, horizontal shift register, column amplifiers to remove fixed pattern noise and a section generating the required frame and line pulses controlled by an on-chip oscillator. Since the area of the chip is extremely small, it is not possible to incorporate more sophisticated features such as programmable gain, offset adjustment, internal automatic gain control etc. on the chip. Instead, the more sophisticated control is preferably implemented by use of a separate controller located remote from the sensor and connected by wires passing along the endoscope. This subdivision requires bi-directional communication between the controller and the imaging arrangement, as will be discussed further below.

As discussed above, light from a point source is radiant on a surface in front of the source with a flux density that varies inversely as the square of the distance between the source and the surface. For instance, the difference between the flux density radiated on two surfaces, one located 1 mm from the source and the other 20 mm from the source, is a factor of 400. The dynamic rage of the image sensors is finite. Often, when an image combines areas of very high brightness and very low brightness, the result is either saturation of the brighter areas or poor display of the darker areas. A miniature endoscope located in a very small intra-bodily tube, for instance the small bronchi, essentially suffers from this effect. The image combines very close and very far portions of tissue, from adjacent tissue at the side to 25-30 mm along the center of the tube. Consequently there is very big difference between the light reflected from the adjacent tissue and the far portions. An image of such tube necessarily suffers from either saturation of the very proximal tissue or too poor noisy quality of display of the dark areas at the center of that tube.

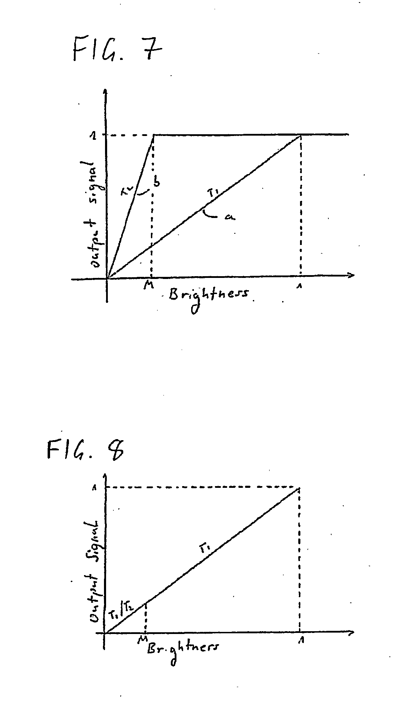

To address this problem, each video frame is preferably exposed with two different exposure durations. Figure 7 shows the output response of two such exposures. For a scene containing a wide range of brightness levels, a sufficiently short exposure duration T₁ avoids saturation over the entire scene. A second T₂ exposure, longer than T₁ by a factor of about 10 times, produces a better image of the darker areas of the image, but gets into saturation for picture elements brighter than some value M. These two images are then combined as shown in figure 8 where the pixels above brightness level M are taken from exposure T₁, and the elements darker than M are taken from exposure T₂ with their values scaled by factor T₁/T₂ to correct for the exposure difference.

In order to achieve color imaging using a monochrome sensor array, the present invention preferably employs a plurality of light sources of different colors, and particularly, red, green and blue (RGB) sources, illustrated here as LEDs **24*a***, **24*b*** and **24*c*.** By capturing frames using sequential illumination by each one of the primary colors alone, the monochrome frames each represent one channel of a color RGB image.

Combining the double exposure technique with the three separate color illuminations yields six exposures for each color frame. Each exposure has its own duration, controlled externally by switching on and off the corresponding illumination source according to exposure control methods to be described below. The pairs of long/short exposures are first combined as described above, The final color image is then the combination of the double exposures for each of the three RGB exposures. First, each of the three basic RGB layers is collected according to the double exposures technique described above. Then the final color image is achieved by chromatic correction done by multiplication of each color layer in the white balance constants. Exact synchronization is needed to switch the LEDs on and off to get homogenous exposure over the entire frame each of the entire frame and to avoid mixing of colors between frames. A rolling-shutter read cycle is preferably triggered between exposures, i.e., when the scene is dark due to lack of illumination, to avoid mixing of the color frames.

Any image sensor suffers to some extent from fixed pattern noise that arises from the variation of the offset and gain of the individual pixels. Data to correct these variations can be measured and stored in a memory, for example an EPROM, implemented as part of the endoscope. Additional distortion may result from any uncorrected chromatic aberrations from lens arrangement **22.** Since every color has its own layer, geometric distortions due to color shifts can be corrected mathematically using geometric transformations. The constants for these transformations can be calibrated individually and stored in the memory of the endoscope.

Turning now to the issue of light leakage and distribution, as a first precaution to minimize light leakage from the light sources directly to the sensor array, the light sources are preferably located as far away from the sensor array as allowed by the dimensions of the imaging arrangement. Thus, in the preferred example shown here (for example in Figure 5), image sensor chip **18** is rectangular, and more particularly square. Light sources **24*a***, **24*b*** and **24*c*** are deployed along no more than two edges of rectangular chip **18,** and sensor array **20** is located proximal to a corner of image sensor chip **18** furthest from the aforementioned two edges.

As a further precaution against light leakage, imaging arrangement **16** preferably further includes a quantity of a substantially opaque medium **28** deployed at least between light sources **24*a***, **24*b*** and **24*c*** and sensor array **20** in such a manner as to avoid obscuring propagation of illumination from the light source towards the scene.

In order to improve uniformity of illumination, and more particularly, to render spatial distribution of light from the different color LEDs more similar, imaging arrangement 16 preferably also includes an optically dispersive medium 30 distally overlying light sources **24*a***, **24*b*** and **24*c*** such that optically dispersive medium **30** is effective to disperse illumination from the light sources **24*a***, **24*b*** and **24*c*,** thereby illuminating the scene viewed from the distal tip portion, without obscuring light reflected from the scene from reaching lens arrangement **22.** This may advantageously be achieved by ensuring that lens arrangement **22** extends distally beyond light sources **24*a***, **24*b*** and **24*c*,** and deploying optically dispersive medium **30** surrounding lens arrangement **30** without overlying the lens. Suitable optically dispersive media include, but are not limited to, adhesives described commercially as "fogged epoxy" and clear adhesives with admixtures of small crystalline or otherwise particulate solids which cause suitable scattering of light.

Optionally, imaging arrangement **16** may further include a substantially transparent medium (not shown) overlying both optically dispersive medium **30** and lens arrangement **22** to encapsulate and protect imaging arrangement **16.**

Preferably, imaging arrangement **16** includes a common circuit board **32** which provides a common mounting structure for light sources **24*a***, **24*b*** and **24*c*** and image sensor chip **18.** Optionally, the light sources may be raised above the surface level of the circuit board by use of a support block not shown) in order to reduce or avoid casting of an illumination shadow by the lens assembly. Circuit board **32** preferably fits within a circular cross-section of diameter 2 millimeters. Most preferably, a roughly circular circuit board of diameter no more than about 1.8 millimeters is used. This facilitates construction of an endoscope with an external diameter no greater than about 2 millimeters.

Electrical wires **42** for supplying power to light sources **24*a***, **24*b*** and **24*c*** and for power supply and data transfer to and from image sensor chip **18** pass along elongated flexible body **12.** In order to facilitate connection of these wires to their respective devices without taking up valuable surface space on the top of the circuit board, connections of the wires are preferably achieved via connection to contact regions of the circuit board on a proximal side of the circuit board, i.e., facing away from the viewing direction. Connections between these contact regions and the components on the circuit board are achieved via through-bores in the circuit board, as is known in the art. Alignment of the wires for attachment to the corresponding contact pads may be achieved using various techniques. By way of one particularly preferred but non-limiting example illustrated in Figure 4, the wires are held in the required formation by a positioning disc **34** configured to leave a small unclad length of each wire projecting. An adapter block **36** is formed with peripheral channels within which the ends of the wires are attached with a small drop of conductive adhesive or solder. The peripheral channels are formed with conductive coatings which are electrically connected to contact pads **38.** Contact pads **38** are deployed so as to align with corresponding contact regions on the rear face of circuit board **32.** Adapter block **36** and circuit board **32** are typically connected with a drop conductive adhesive or solder applied to each of contact pads **38.**

Preferably, distal tip portion **14** includes a position sensor arrangement **40** (Figure 2), including a plurality of sensor coils, deployed near a proximal side of the circuit board. Position sensor arrangement **40** is preferably implemented as a sensor arrangement of a six-degrees-of-freedom position measurement system, and most preferably according to the teachings of U.S. Patent No. 6,188,355 and published PCT Application Nos. WO 00/10456 and WO 01/67035, all of which are hereby incorporated by reference. The position sensor arrangement **40** provides tracking of the position of imaging arrangement **16** within the body, thereby facilitating navigation of the endoscope and integration of the imaging data with other available sources of information.

As mentioned above, one of the factors problematic for miniaturization of an endoscope is the number of wires **42** which extend along the flexible body and must be connected to the imaging arrangement. In order to reduce the number of wires as much as possible, it is a particularly preferred feature of certain embodiments of the present invention that image sensor chip **18** is connected to exactly four wires. Operation of imaging arrangement **16** is controlled by a controller, which may be implemented as a dedicated electronics unit or as part of a general purpose computer system **44** (Figure 1), associated with a proximal part of elongated flexible body **12.** In order to achieve four-wire connection of the image sensor chip, the controller is electrically associated with image sensor chip **18** via no more than two communication wires **42** extending along the elongated flexible body **12.** Communication is preferably achieved bi-directionally, by configuring image sensor chip **18** to be responsive to a frame request signal generated by the controller to perform a read cycle of the two-dimensional array of light-sensitive pixels in a rolling-shutter mode, and to transmit a single frame of image data to the controller. Both the frame request signal and the image data are transmitted via one or both of the two communication wires. Preferably, at least the image data is transmitted on both wires using a differential signal in order to minimize data corruption.

Synchronization of the read cycle of image sensor chip **18** is preferably controlled by the controller. Thus, image sensor chip **18** is preferably configured to wait after transmitting the single frame of image data until receiving a subsequent frame request signal from the controller. In practice, since the imaging arrangement operates in darkness, exposure control is preferably primarily achieved by controlling the activation time of the illumination sources, also controlled by the controller. However, for efficient use of time, it is preferable that a read cycle of the chip is initiated immediately after each exposure is completed. Thus, the controller is preferably configured to generate frame request signals at the end of pairs of unequal periods, corresponding to the aforementioned short and long exposure times for each color illumination. As explained earlier, pairs of similar frames with different exposure durations are generated, and are co-processed by the controller to derive an enhanced frame having a dynamic range greater than each of the pair of similar frames. In the aforementioned preferred color imaging implementation, the controller is preferably also configured to combine the enhanced frames for each of the three colors of illumination to generate a color image.

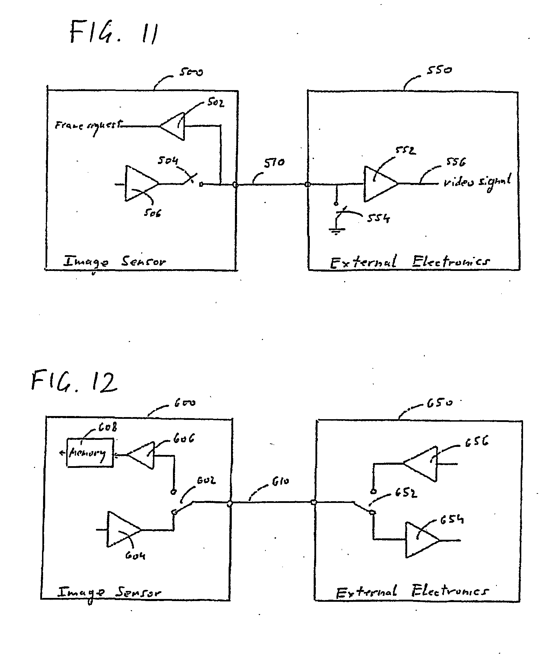

One non-limiting example of a simple electronic implementation for the bidirectional communication between image sensor chip **18** and the controller is illustrated in Figure 11. The electronics of image sensor chip **18** is here designated **500** while the external electronics (part of the controller) is designated **550.** The image sensor chip operates in rolling shutter mode with external synchronization via a frame request from the controller transmitted along the video output line **510,** which is preferably a dual differential line.

The video out signal, either digital or analog, is derived by driver **506** and transmitted through the closed switch **504** and along line **510** to a receiver **552,** which delivers the video signal **556** to its final destination (e.g., computer system **44** or any other required equipment). After a full frame is transferred, switch **504** is opened, and the image sensor waits for a frame request command. In this state, the cells collect photon electrons. For actuating a frame request, switch **554** is closed, grounding line **510,** and thereby changing the output of amplifier **502** so as to activate the next read cycle of the image array. The read cycle also resets the pixels during the image output data transfer, row by row. The external electronics **550** can be controlled by a PC, micro-controller or any other suitable state machine.

A second non-limiting example of a more sophisticated architecture is described in figure 12. Here, the image sensor electronics **600** and external electronics **650** are connected via a bi-directional communication line **610,** again preferably a dual differential line. During the video image data transmission, a first switch **602** connects the output driver **604** to line **610** and a second switch **652** connects line **610** to an amplifier **654** to receive the signal. After completion of transfer of a frame, switches **602** and **652** change state, allowing driver **656** to send digital data to the image sensor, received by amplifier **606** and stored into memory **608.** This digital data is used to control the delay between sequential video frames using count down counter. Optionally this port can serve also for other control commands.

In either of the above cases, a practical implementation of the electronic arrangement is well within the capabilities of one ordinarily skilled in the art and will not be addressed here in further detail.

Turning finally to Figures 6A-6C, there is shown an apparatus **200** useful for assembly of imaging arrangement **16,** and in particular, for correctly aligned attachment of lens arrangement **22** by clear adhesive to the sensor array of chip **18.** Apparatus **200** has a first adjustable platform **202** with a clamping surface **204** (Figure 6B) for gripping circuit board **32** which carries image sensor chip **18.** Adjustable platform **202** is mounted beneath a microscope **206** so that chip **18** can be viewed and so that the sensor array **20** can be centered under a reticule of microscope **206** by adjustment of platform **202.** Apparatus **200** further includes an adjustable support **208** to which a hinged flap **210** is hingedly mounted. Hinged flap **210** includes a lens holder **212** for clamping lens arrangement **22** in a well-defined centered position. Adjustable support **208** also allows adjustment of the position of hinged flap **210** for centering a marker on the rear (upper) side of lens holder **212** relative to the microscope reticule when the flap is in its lowered position (Figure 6C).

Before use, hinged flap **210** is lowered to the position of Figure 6C and adjustable support **208** is adjusted until lens holder **212** is centered relative to the microscope reticule. Flap **210** is then raised to the position of Figure 6A, and lens arrangement **22** is inserted into lens holder **212.** Circuit board **32** is clamped to clamping surface **204** and adjustable platform **202** is adjusted to center the sensor array **20** relative to the microscope reticule. A small quantity of clear adhesive is then applied to the end of lens arrangement **22** and hinged flap **210** is gently lowered to bring lens arrangement **22** into contact with the sensor array where it is left until dry. Hinged flap **210** is preferably configured to apply a predefined contact pressure between the lens arrangement and the sensor array, thereby helping to ensure that the lens arrangement seats itself squarely against the chip surface.

It will be appreciated that the above descriptions are intended only to serve as examples, and that many other embodiments are possible within the scope of the present invention as defined in the appended claims.

## Claims

1. An endoscope (10) comprising:
(a) an elongated flexible body (12) having a distal tip portion (14);
(b) at least one light source (24a, 24b, 24c) for illuminating the scene viewed from said distal tip portion;
(c) an optically dispersive medium (30) distally overlying said light source such that said optically dispersive medium is effective to disperse illumination from said light source, thereby illuminating the scene viewed from said distal tip portion, without obscuring light reflected from the scene from reaching said lens arrangement;
(d) an imaging arrangement (16) associated with said distal tip portion, said imaging arrangement including:
(i) an image sensor chip (18) including a two-dimensional array (20) of light-sensitive pixels; and
(ii) a lens arrangement (22) deployed for focusing light from a field of view onto said image sensor chip so as to generate an image of a scene viewed from said distal tip portion, wherein
said lens arrangement is directly affixed to said image sensor chip by a quantity of transparent adhesive, **characterized in that**;
said lens arrangement extends distally beyond said at least one light source, and wherein said optically dispersive medium surrounds said lens arrangement without overlying said lens arrangement.

2. The endoscope of claim 1, wherein said lens arrangement includes a cylindrical graded-index lens.

3. The endoscope of claim 1, wherein said lens arrangement includes a compound lens assembly.

4. The endoscope of claim 1, wherein said lens arrangement has a field of view of at least about 60[deg.].

5. The endoscope of claim 1, wherein said lens arrangement has a field of view of at least about 90[deg.].

6. The endoscope of claim 1, wherein an area of said two-dimensional array of light-sensitive pixels is no more than half a square millimeter.

7. The endoscope of claim 1, wherein said imaging arrangement has a diameter of no more than 2 millimeters.

8. The endoscope of claim 1, wherein said imaging arrangement further includes a substantially opaque medium deployed at least between said light source and said two-dimensional array of light-sensitive pixels without obscuring propagation of illumination from said light source towards the scene.

9. The endoscope of claim 1, wherein said imaging arrangement further includes a substantially transparent medium overlying both said optically dispersive medium and said lens arrangement.

10. The endoscope of claim 1, wherein said at least one light source is implemented as a plurality of light sources of different colors.

11. The endoscope of claim 10, wherein said image sensor chip is rectangular, and wherein said plurality of light sources are deployed along no more than two edges of said rectangular chip, said two-dimensional array of light-sensitive pixels being located proximal to a corner of said image sensor chip furthest from said two edges of said rectangular chip.

12. The endoscope of claim 1, wherein said at least one light source and said image sensor chip are deployed on a common circuit board.

13. The endoscope of claim 12, wherein said circuit board fits within a circular cross-section of diameter 2 millimeters.

14. The endoscope of claim 12, further comprising a plurality of wires passing along said elongated flexible body for connection to said image sensor chip and said at least one light source, said wires being connected to contact regions of said circuit board on a proximal side of said circuit board.

15. The endoscope of claim 14, wherein said image sensor chip is connected to exactly four of said plurality of wires.

16. The endoscope of claim 12, further comprising a position sensor arrangement (40) including a plurality of sensor coils, said position sensor arrangement being deployed within said elongated flexible body near a proximal side of said circuit board.

## Patentansprüche

1. Endoskop (10), umfassend:
(a) länglichen flexiblen Körper (12), welcher einen distalen Spitzen-Abschnitt (14) aufweist;
(b) mindestens eine Lichtquelle (24a, 24b, 24c) zur Ausleuchtung des Ansichtsbereiches, welcher von dem distalen Spitzen-Abschnitt aus betrachtet wird;
(c) optisch dispersives Medium (30), welches die Lichtquelle in distaler Richtung auf solch eine Weise überlagert, dass das optisch dispersive Medium effektiv eingesetzt wird, um das von der Lichtquelle kommende Licht zu zerstreuen, wodurch der Ansichtsbereich beleuchtet wird, welcher von dem distalen Spitzen-Abschnitt aus betrachtet wird, ohne dabei das Licht zu schwächen, welches von dem Ansichtsbereich reflektiert wird, und [es daran zu hindern], die Linsenanordnung zu erreichen;
(d) Abbildungsanordnung (16), welche mit dem distalen Spitzen-Abschnitt verbunden ist, wobei die Abbildungsanordnung umfasst:
(i) Bildsensor-Chip (18), welcher eine zweidimensionale Anordnung (20) aus lichtempfindlichen Pixels umfasst; und
(ii) Linsenanordnung (22), die eingesetzt wird, um Licht aus einem Sichtfeld auf den Bildsensor-Chip zu bündeln, so dass ein Bild eines Ansichtsbereiches generiert wird, welcher von dem distalen Spitzen-Abschnitt aus betrachtet wird, wobei
die Linsenanordnung mittels einer Menge an transparentem Klebstoff direkt an dem Bildsensor-Chip angebracht ist, **dadurch gekennzeichnet, dass**;
die Linsenanordnung sich in distaler Richtung bis jenseits der mindestens einen Lichtquelle erstreckt, und wobei das optisch dispersive Medium die Linsenanordnung umgibt, ohne die Linsenanordnung zu überlagern.

2. Endoskop gemäß Anspruch 1, wobei die Linsenanordnung eine zylindrische Gradientenindexlinse umfasst.

3. Endoskop gemäß Anspruch 1, wobei die Linsenanordnung eine Verbundlinsen-Anordnung umfasst.

4. Endoskop gemäß Anspruch 1, wobei die Linsenanordnung ein Sichtfeld von mindestens 60 [Grad] aufweist.

5. Endoskop gemäß Anspruch 1, wobei die Linsenanordnung ein Sichtfeld von mindestens 90 [Grad] aufweist.

6. Endoskop gemäß Anspruch 1, wobei die Fläche der aus lichtempfindlichen Pixels bestehenden zweidimensionalen Anordnung nicht mehr als einen halben Quadratmillimeter beträgt.

7. Endoskop gemäß Anspruch 1, wobei die Abbildungsanordnung einen Durchmesser von nicht mehr als 2 Millimetern hat.

8. Endoskop gemäß Anspruch 1, wobei die Abbildungsanordnung ferner ein in wesentlichen strahlenundurchlässiges Medium umfasst, welches mindestens zwischen der Lichtquelle und der aus lichtempfindlichen Pixels bestehenden zweidimensionalen Anordnung zum Einsatz kommt, ohne dabei die Ausbreitung des Lichts von der Lichtquelle zu der Ansicht hin zu beeinträchtigen.

9. Endoskop gemäß Anspruch 1, wobei die Abbildungsanordnung ferner ein im wesentlichen transparentes Medium umfasst, welches sowohl das optisch dispersive Medium als auch die Linsenanordnung überlagert.

10. Endoskop gemäß Anspruch 1, wobei die mindestens eine Lichtquelle in Form einer Vielzahl von Lichtquellen verschiedener Farben implementiert ist.

11. Endoskop gemäß Anspruch 10, wobei der Bildsensor-Chip rechteckig ist, und wobei die Vielzahl von Lichtquellen entlang nicht mehr als zwei Rändern des rechteckigen Chip zum Einsatz kommt, wobei die aus lichtempfindlichen Pixels bestehende zweidimensionale Anordnung proximal zu einer Ecke des Bildsensor-Chip angeordnet ist, welche sich am weitesten entfernt von den zwei Kanten des rechteckigen Chip befindet.

12. Endoskop gemäß Anspruch 1, wobei die mindestens eine Lichtquelle und der Bildsensor-Chip auf einer gemeinsamen Leiterplatte zum Einsatz kommen.

13. Endoskop gemäß Anspruch 12, wobei die Leiterplatte in einen runden Querschnitt mit einem Durchmesser von 2 Millimetern hineinpasst.

14. Endoskop gemäß Anspruch 12, ferner eine Vielzahl von Drähten umfassend, welche entlang des länglichen flexiblen Körpers verlaufen, so dass eine Verbindung zu dem Bildsensor-Chip und der mindestens einen Lichtquelle zustande kommt, wobei die Drähte an Kontaktbereiche der Leiterplatte angeschlossen sind, welche sich an einer proximalen Seite der Leiterplatte befinden.

15. Endoskop gemäß Anspruch 14, wobei der Bildsensor-Chip mit exakt vier der Vielzahl von Drähten verbunden ist.

16. Endoskop gemäß Anspruch 12, ferner eine Positionssensor-Anordnung (40) umfassend, welche eine Vielzahl von Sensorspulen umfasst, wobei die Positionssensor-Anordnung innerhalb des länglichen flexiblen Körpers nahe einer proximalen Seite der Leiterplatte zum Einsatz gebracht wird.

## Revendications

1. Endoscope (10) comprenant :
(a) un corps souple allongé (12) ayant une partie d'extrémité distale (14),
(b) au moins une source de lumière (24a, 24b, 24c) destinée à illuminer la scène observée depuis ladite partie d'extrémité distale,
(c) un milieu de dispersion optique (30) recouvrant de manière distale ladite source de lumière de sorte que ledit milieu de dispersion optique soit efficace pour disperser l'illumination provenant de ladite source de lumière, en illuminant de cette manière la scène observée depuis ladite partie d'extrémité distale, sans empêcher la lumière réfléchie depuis la scène d'atteindre ledit agencement d'objectif,
(d) un agencement de formation d'images (16) associé à ladite partie d'extrémité distale, ledit agencement de formation d'images incluant :
(i) une puce de capteur d'image (18) incluant une matrice à deux dimensions (20) de pixels sensibles à la lumière, et
(ii) un agencement d'objectif (22) déployé pour focaliser la lumière provenant d'un champ de vision sur ladite puce de capteur d'image de manière à générer une image de la scène observée depuis ladite partie d'extrémité distale, dans lequel
ledit agencement d'objectif est directement fixé sur ladite puce de capteur d'image par une certaine quantité d'un élément adhésif transparent, **caractérisé en ce que**,
ledit agencement d'objectif s'étend de manière distale au-delà de ladite au moins une source de lumière, et dans lequel ledit milieu de dispersion optique entoure ledit agencement d'objectif sans recouvrir ledit agencement d'objectif.

2. Endoscope selon la revendication 1, dans lequel ledit agencement d'objectif inclut une lentille à gradient d'indice cylindrique.

3. Endoscope selon la revendication 1, dans lequel ledit agencement d'objectif inclut un ensemble d'objectif composé.

4. Endoscope selon la revendication 1, dans lequel ledit agencement d'objectif a un champ de vision d'au moins environ 60 degrés.

5. Endoscope selon la revendication 1, dans lequel ledit agencement d'objectif a un champ de vision d'au moins environ 90 degrés.

6. Endoscope selon la revendication 1, dans lequel une surface de ladite matrice à deux dimensions de pixels sensibles à lumière n'est pas supérieure à la moitié d'un millimètre carré.

7. Endoscope selon la revendication 1, dans lequel ledit agencement de formation d'images a un diamètre pas supérieur à 2 millimètres.

8. Endoscope selon la revendication 1, dans lequel ledit agencement de formation d'images inclut en outre un milieu globalement opaque déployé entre ladite source de lumière et ladite matrice à deux dimensions de pixels sensibles de lumière sans empêcher la propagation de l'illumination provenant de la source de lumière en direction de la scène.

9. Endoscope selon la revendication 1, dans lequel ledit agencement de formation d'images inclut en outre un milieu globalement transparent recouvrant à la fois ledit milieu de dispersion optique et ledit agencement d'objectif.

10. Endoscope selon la revendication 1, dans lequel au moins une source de lumière est mise en oeuvre sous la forme d'une pluralité de sources de lumière de différentes couleurs.

11. Endoscope selon la revendication 10, dans lequel ladite puce de capteur d'image est rectangulaire, et dans lequel ladite pluralité de sources de lumière sont déployées le long de pas plus que deux bords de ladite puce rectangulaire, ladite matrice à deux dimensions de pixels sensibles à la lumière étant située à proximité d'un coin de la puce de capteur d'image le plus éloigné desdits deux bords de ladite puce rectangulaire.

12. Endoscope selon la revendication 1, dans lequel ladite au moins une source de lumière et ladite puce de capteur d'image sont déployées sur une carte de circuit commune.

13. Endoscope selon la revendication 12, dans lequel ladite carte de circuit loge dans une section transversale circulaire de 2 millimètres de diamètre.

14. Endoscope selon la revendication 12, comprenant en outre une pluralité de fils passant le long dudit corps souple allongé pour une connexion à ladite puce de capteur d'image et à ladite au moins une source de lumière, lesdits fils étant connectés à des régions de contact de ladite carte de circuit sur un côté proximal de ladite carte de circuit.

15. Endoscope selon la revendication 14, dans lequel ladite puce de capteur d'image est connectée à exactement quatre fils de ladite pluralité de fils.

16. Endoscope selon la revendication 12, comprenant en outre un agencement de capteur de position (40) incluant une pluralité de bobines de capteurs, ledit agencement de capteur de position étant déployé dans ledit corps souple allongé à proximité d'un côté proximal de ladite carte de circuit.
